(19) Europäisches Patentamt
European Patent Office
Office européen des brevets

(11) Veröffentlichungsnummer: **0 062 874**
**B1**

(12) **EUROPÄISCHE PATENTSCHRIFT**

(45) Veröffentlichungstag der Patentschrift: **24.10.84**

(51) Int. Cl.³: **C 07 C 69/60,** C 07 C 67/08

(21) Anmeldenummer: **82102894.1**

(22) Anmeldetag: **05.04.82**

# (54) Verfahren zur Herstellung von Maleinsäuredialkylestern.

(30) Priorität: **09.04.81 DE 3114320**

(43) Veröffentlichungstag der Anmeldung: **20.10.82 Patentblatt 82/42**

(45) Bekanntmachung des Hinweises auf die Patenterteilung: **24.10.84 Patentblatt 84/43**

(84) Benannte Vertragsstaaten: **BE CH DE FR GB IT LI NL**

(56) Entgegenhaltungen:
**GB - A - 1 437 898**
**US - A - 3 078 302**
**US - A - 4 032 458**

**Fleet and Gardner: Maleic Anhydride Derivatides New York (1952)**

(73) Patentinhaber: **HOECHST AKTIENGESELLSCHAFT, Postfach 80 03 20, D-6230 Frankfurt am Main 80 (DE)**

(72) Erfinder: **Günther, Klaus, Dr., Waldstrasse 5, D-6239 Eppstein/Taunus (DE)**
Erfinder: **Wendt, Heinz, Dr., Kronberger Weg 20, D-6231 Sulzbach (DE)**

**EP 0 062 874 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Maleinsäuredialkylestern

$$\begin{array}{c} HC-COOR \\ \| \\ HC-COOR \end{array}$$

wobei $R=CH_3$, $C_2H_5$ oder $C_3H_7$ sein kann, durch Veresterung von Maleinsäure oder Maleinsäureanhydrid oder deren Gemischen mit aliphatischen Alkoholen ROH, wobei R die eben genannte Bedeutung hat, in Gegenwart eines Katalysators.

Die genannten Maleinsäuredialkylester, im folgenden meist kurz als der Methylester, der Ethylester, der Propylester bzw. der Isopropylester bezeichnet, werden technisch als Zwischenprodukte z.B. zur Herstellung von Copolymerisaten für Dispersion eingesetzt, die in Anstrichen oder Leimen verwendet oder auch zur Herstellung von Filmen und Folien.

Die Maleinsäuredialkylester gehen die für Verbindungen mit olefinischen Doppelbindungen üblichen Anlagerungsreaktionen ein und eignen sich z.B. als Dienophil für Diensynthesen nach Diels-Adler. Durch Hydrierung oder Acetylierung erhält man wertvolle Zwischenprodukte, z.B. Bernstein-Säureester und deren Derivate, die auf den verschiedensten Gebieten der organischen Synthesen Verwendung finden.

Die Herstellung der genannten Maleinsäuredialkylester durch Veresterung von Maleinsäure oder Maleinsäureanhydrid mit den entsprechenden Alkoholen ist bereits bekannt. Sie wird in Gegenwart eines Katalysators durchgeführt, der im allgemeinen sauer ist. In erster Linie wird als Katalysator Schwefelsäure verwendet. Daneben sind auch andere anorganische Säuren, wie Salzsäure oder Phosphorsäure, und Arylsulfonsäuren, wie Toluolsulfonsäure, geeignet. Die Säurereste können auch an Trägern fixiert sein, wie z.B. bei sauren Austauscherharzen.

Die Veresterung wird bisher technisch sowohl diskontinuierlich als auch kontinuierlich unter Normaldruck durchgeführt. In beiden Fällen wird das bei der Veresterung gebildete Wasser durch Destillation aus dem Gleichgewicht entfernt, wobei jedoch Alkohol mitübergeht. Da jedoch der Alkohol mit Wasser in jedem Verhältnis mischbar ist und deswegen nach der Kondensation des Wassers keine Phasentrennung eintritt, setzt man, falls möglich, vorteilhafterweise eine geeignete Hilfsflüssigkeit zu, die eine Phasentrennung bewirkt. Als derartige Hilfsflüssigkeiten benutzt man solche Substanzen, die mit Wasser relativ niedrig siedende Azeotrope mit hohem Wassergehalt bilden. Für die Gewinnung des Ethyl-, Propyl- und Isopropylesters sind als Hilfsflüssigkeiten z.B. Benzol, Diisopropylether oder Chloroform geeignet. Die organische Phase wird dabei nach der Abtrennung in das Reaktionsgefäss zurückgeführt und wirkt hier wieder als Schleppmittel für das Reaktionswasser. Das abgetrennte Wasser wird einer gesonderten Aufarbeitung zugeführt, um gelöste geringe Mengen Alkohol und Schleppmittel zurückzugewinnen. Bei der Gewinnung des Methylesters setzt man im allgemeinen kein Schleppmittel ein, sondern entfernt das Reaktionswasser durch gemeinsames Abdestillieren mit überschüssigem Methanol, wobei das entstehende wässerige Methanol in einer separaten Anlage entwässert wird. Bei der diskontinuierlichen Veresterung von Maleinsäure bzw. Maleinsäureanhydrid besteht die Apparatur im wesentlichen aus einem ausreichend grossen Reaktionsgefäss mit aufgesetzter kleiner Kolonne, Kondensator und einem Wasserabscheider. Das Reaktionsgemisch aus Maleinsäure bzw. Maleinsäureanhydrid, Alkohol, Katalysator und ggf. Schleppmittel wird im Reaktionsgefäss erhitzt und bei Gegenwart eines Schleppmittels das Reaktionswasser aus dem Abscheider abgezogen, bzw. bei Abwesenheit eines Schleppmittels der kondensierte wässerige Alkohol destillativ aufgearbeitet. Um den Veresterungsprozess möglichst weitgehend in Richtung auf die Esterbildung zu verschieben, setzt man einen Überschuss an Alkohol zu. Der Fortgang der Veresterung wird durch den im Reaktionsgefäss vorhandenen Restsäuregehalt kontrolliert. Nach Beendigung der Veresterung destilliert man den verbliebenen Alkohol ab und gewinnt so den Rohester, der in üblicher Weise, d.h. durch Neutralisation der Restsäure, Trocknung und Reindestillation, zum Reinester aufgearbeitet wird.

Bei der kontinuierlichen Arbeitsweise führt man das Gemisch aus Maleinsäure bzw. Maleinsäureanhydrid und dem eingesetzten Alkohol zusammen mit dem Katalysator auf den Kopf einer geeigneten Bodenkolonne, die als Reaktor dient, und destilliert ggf. vom unteren Teil der Kolonne ein Schleppmittel durch die Kolonne. Das Veresterungswasser wird in Form von wässerigem Alkohol am Kopf der Kolonne abgenommen, bzw. zusammen mit dem Schleppmittel in einem Abscheider in die organische Phase und die wässerige Phase getrennt. Aus dem Sumpf der Reaktionskolonne zieht man den Rohester ab. Die weitere Aufarbeitung verläuft wie beim diskontinuierlichen Verfahren.

Bei grösseren Anlagen zur kontinuierlichen Herstellung des Ethylesters kann man auch auf den Einsatz des Schleppmittels verzichten, indem man z.B. Maleinsäureanhydrid und Ethanol in einer Kolonne im Gegenstrom fährt und das Reaktionswasser am Kopf der Veresterungskolonne zusammen mit überschüssigem Ethanol abzieht. Das wässerige Ethanol wird dann in einer separaten Anlage entwässert, z.B. in einer 2-Stufen-Druckdestillation.

Sowohl dem diskontinuierlichen als auch dem kontinuierlichen Verfahren ist gemeinsam, dass die Verweilzeit des Reaktionsgemisches in der Reaktionszone unter den Reaktionsbedingungen relativ hoch sein muss, um einen ausreichenden Veresterungsgrad zu erzielen. Die erwähnten Reaktionsbedingungen werden so gewählt, dass einmal ein Alkoholüberschuss von bis zu 200%, bezogen auf die stöchiometrische Menge (Überschuss von 200% bedeutet 6 mol Alkohol pro Mol Maleinsäure) im Reaktionsgemisch vorhanden ist, zum anderen muss die Reaktionstemperatur einen für eine sinnvolle Reaktionsgeschwindigkeit entsprechenden Wert haben. Beide Parameter haben jedoch einen gegenläufigen Einfluss, d.h. bei hohem Alkoholüberschuss ergibt sich wegen der relativ niedrigen Siedepunkte der eingesetzten Alkohole eine relativ niedrige Veresterungstemperatur; eine höhere Veresterungstemperatur kann man erzielen, wenn der Alkoholüberschuss im Reaktionsgemisch vermindert wird. Andererseits ist bekannt, dass eine höhere Veresterungstemperatur in erheblichem Umfang eine Umlagerung des Maleinsäureesters in den thermodynamisch stabileren, isomeren Fumarsäureester zur Folge hat.

Daher hat man bisher im allgemeinen bei der Herstellung der einzelnen Ester Reaktionstemperaturen eingestellt, die 10 bis 50°C über den Siedepunkten der eingesetzten Alkohole lagen. Diese Siedepunkte haben folgende Werte: Methylalkohol 65°C, Ethylalkohol 78°C, Propylalkohol 97°C, Isopropylalkohol 82°C. Bei den genannten Reaktionstemperaturen wird zur vollständigen Veresterung eine Zeitspanne von 26 bis 48 h benötigt. Unter diesen Reaktionsbedingungen erhält man in dem rohen Maleinsäureester einen Gehalt an Fumarsäureester von ca. 4 bis 9 Gew.-%. Diese Fumaresterkonzentration ist jedoch für viele Anwendungszwecke unerwünscht. Es ist unter den genannten Bedingungen praktisch jedoch nicht möglich, einen Maleinsäureester herzustellen, der einen Fumarestergehalt von weniger als 4 Gew.-% aufweist.

Überraschenderweise wurde nun gefunden, dass ein Maleinsäureester mit einem Fumarestergehalt unter 4 Gew.-% erhalten wird, wenn man die Veresterung unter Druck bei erhöhten Temperaturen durchführt. Das erfindungsgemässe Verfahren zur Herstellung von Maleinsäuredialkyl-

HC−COOR
estern ‖ , wobei R=$CH_3$, $C_2H_5$ oder
HC−COOR

$C_3H_7$ sein kann, durch Veresterung von Maleinsäure oder Maleinsäureanhydrid oder deren Gemischen mit aliphatischen Alkoholen ROH, wobei R die eben genannte Bedeutung hat, in Gegenwart eines Katalysators, ist dadurch gekennzeichnet, dass man die Veresterung mit einem Alkoholüberschluss bis zu 300%, bezogen auf die Stöchiometrie, unter einem Überdruck von 1 bis 20 bar und bei einer Reaktionstemperatur durchführt, die 60 bis 100°C über dem Siedepunkt des eingesetzten Alkohols liegt.

Vorzugsweise ist der Alkoholüberschuss 20 bis 200, insbesondere 50 bis 150%, bezogen auf die Stöchiometrie. Der Überdruck ist vorzugsweise 1 bis 10, insbesondere 2 bis 3 bar. Beim erfindungsgemässen Verfahren sind merklich kürzere Veresterungszeiten erforderlich als nach dem Stand der Technik. Trotz der relativ hohen Reaktionstemperatur erhält man ein Produkt mit sehr niedrigem Gehalt an Fumarester. Dieser Effekt war nicht zu erwarten, denn es ist bekannt, dass Maleinsäureester leicht durch Erhitzen in die entsprechenden Fumarester umgewandelt werden können. Die erfindungsgemässe Druckveresterung kann sowohl bei der diskontinuierlichen als auch bei der kontinuierlichen Arbeitsweise angewendet werden.

Bei der diskontinuierlichen Veresterung ordnet man das Druckregelventil vorzugsweise so, an dass sowohl das Reaktionsgefäss als auch die Kolonne, der Kondensator und der Wasserabscheider unter dem erhöhten Druck stehen.

Bei der kontinuierlichen Veresterung wird vorzugsweise nur der Reaktor unter erhöhtem Druck betrieben.

*Beispiel*

Als Reaktor dient eine mit 25 Siebböden ausgestattete Kolonne aus Edelstahl, die einen Innendurchmesser von 150 mm sowie eine Höhe von ca. 3000 mm aufweist. Die Kolonne ist mit einer regelbaren elektrischen Heizung versehen; die Temperatur wird auf ca. 160°C eingestellt.

Unterhalb des obersten Bodens der Kolonne werden 1,73 kg/h Ethanol und 0,02 kg/h Maleinsäureanhydrid, 0,74 kg/h konzentrierte Schwefelsäure eingespeist (Molverhältnis Maleinsäureanhydrid/Ethanol = 1:1). Oberhalb des untersten Kolonnenbodens werden, 2,80 kg/h wasserfreies, auf 160°C vorgeheitzes Ethanol eingespeist. Die insgesamt eingesetzte Ethanolmenge liegt 120% über der stöchiometrisch notwendigen Menge. Der Überdruck in der Kolonne beträgt 3 bar.

Das Kopfprodukt der Kolonne wird auf Atmosphärendruck entspannt und abgekühlt. Es fällt in einer Menge von 2,10 kg/h an und besteht aus 85,2 Gew.-% Ethanol und 14,8 Gew.-% Wasser.

Aus dem Sumpf der Kolonne werden 3,17 kg/h Produkt entnommen, dessen Zusammensetzung wie folgt ist: 84,5 Gew.-% Diethylmaleinat, 3,2 Gew.-% Diethylfumarat, 7,9 Gew.-% Ethanol, 3,8 Gew.-% Sonstige, 0,6 Gew.-% saure Komponenten.

Die Verweilzeit beträgt 19 h, bezogen auf Maleinsäuremonoethylester.

**Patentansprüche**

1. Verfahren zur Herstellung von Maleinsäure-

HC−COOR
dialkylestern der Formel ‖ , wobei
HC−COOR

R=$CH_3$, $C_2H_5$ oder $C_3H_7$ sein kann, durch Veresterung von Maleinsäure oder Maleinsäureanhydrid oder deren Gemischen mit aliphatischen Alkoholen ROH, wobei R die eben genannte Bedeutung hat, in Gegenwart eines Katalysators, dadurch gekennzeichnet, dass man die Veresterung mit einem Alkoholüberschuss bis zu 300%, bezogen auf die Stöchiometrie, unter einem Überdruck von 1 bis 20 bar bei einer Reaktionstemperatur durchführt, die 60 bis 100°C über dem Siedepunkt des eingesetzten Alkohols liegt.

2. Verfahren nach Anspruch 1, dadurch gekennzeichnet, dass man die Reaktion unter einem Überdruck von 1 bis 10 bar durchführt.

3. Verfahren nach Anspruch 1, dadurch gekennzeichnet, dass man die Reaktion unter einem Überdruck von 2 bis 3 durchführt.

4. Verfahren nach einem der Ansprüche 1 bis 3, dadurch gekennzeichnet, dass man die Reaktion

bei einer Temperatur durchführt, die 70 bis 90°C über dem Siedepunkt des eingesetzten Alkohols liegt.

5. Verfahren nach einem der Ansprüche 1 bis 4, dadurch gekennzeichnet, dass man die Reaktion mit einem Alkoholüberschuss von 20 bis 200%, bezogen auf die Stöchiometrie, durchführt.

6. Verfahren nach einem der Ansprüche 1 bis 4, dadurch gekennzeichnet, dass man die Reaktion mit einem Alkoholüberschuss von 50 bis 150%, bezogen auf die Stöchiometrie, durchführt.

## Claims

1. A process for the manufacture of dialkyl maleates of the formula

$$\begin{array}{l} HC-COOR \\ \| \\ HC-COOR \end{array}$$

, in which R is $CH_3$, $C_2H_5$ or $C_3H_7$ by esterification of maleic acid or maleic anhydride or mixtures thereof, with aliphatic alcohols of the formula ROH, in which R is defined as above, in the presence of a catalyst, which is characterized by carrying out the esterification by using an excess of alcohol up to 300%, referred to the stoichiometric quantity, under a pressure from 1 to 20 bar gauge and at a reaction temperature by 60 to 100°C above the boiling point of the alcohol used.

2. The process of Claim 1, wherein the reaction is carried out under a pressure of from 1 to 10 bar gauge.

3. The process of Claim 1, wherein the reaction is carried out under a pressure of from 2 to 3 bar gauge.

4. The process of any one of Claims 1 to 3, wherein the reaction is carried out at a temperature being 70 to 90°C above the boiling point of the alcohol used.

5. The process of any one of Claims 1 to 4, wherein the reaction is carried out by using an excess of alcohol of from 20 to 200%, referred to the stoichiometric quantity.

6. The process of any one of claims 1 to 4, wherein the reaction is carried out by using an excess of alcohol of from 50 to 150%, referred to the stoichiometric quantity.

## Revendications

1. Procédé de préparation de maléates de dialkyles répondant à la formule

$$\begin{array}{l} HC-COOR \\ \| \\ HC-COOR \end{array}$$

, dans laquelle R représente un radical $CH_3$, $C_2H_5$ ou $C_3H_7$, par estérification de l'acide maléique ou de l'anhydride maléique, ou de mélanges de ces composés, avec des alcools aliphatiques ROH dans lesquels R a la signification qui vient d'être donnée, en présence d'un catalyseur, procédé caractérisé en ce qu'on effectue l'estérification avec un excès d'alcool pouvant aller jusqu'à 300% par rapport à la quantité stœchiométrique, sous une pression effective de 1 à 20 bar et à une température réactionnelle supérieure de 60 à 100°C au point d'ébullition de l'alcool mis en jeu.

2. Procédé selon la revendication 1, caractérisé en ce qu'on effectue la réaction sous une pression effective de 1 à 10 bar.

3. Procédé selon la revendication 1, caractérisé en ce qu'on effectue la réaction sous une pression effective de 2 à 3 bar.

4. Procédé selon l'une des revendications 1 à 3, caractérisé en ce qu'on effectue la réaction à une température supérieure de 70 à 90°C au point d'ébullition de l'alcool mis en jeu.

5. Procédé selon l'une des revendications 1 à 4, caractérisé en ce qu'on effectue la réaction avec un excès d'alcool de 20 à 200% par rapport à la proportion stœchiométrique.

6. Procédé selon l'une des revendications 1 à 4, caractérisé en ce qu'on effectue la réaction avec un excès d'alcool de 50 à 150% par rapport à la proportion stœchiométrique.